# EUROPÄISCHE PATENTANMELDUNG

(11) **EP 3 616 500 A1**
(43) Veröffentlichungstag der Anmeldung: **04.03.2020**
(21) Anmeldenummer: 19193291.2
(22) Anmeldetag: 23.08.2019
(51) Int. Cl.: A01G 17/00, A01G 2/30, A01G 2/00, A01H 6/74

(54) **VERFAHREN ZUR HERSTELLUNG EINES PRÄFORMIERTEN JUNGBAUMS, FÜR ÄPFEL UND BIRNEN, EIN VERFAHREN ZUR ERSTELLUNG VON OBSTPLANTAGEN, UMFASSEND EINEN SOLCHEN JUNGBAUM MIT VERBESSERTEN MERKMALEN FÜR DIE AUTOMATISIERTE MECHANISCHE ERNTE**

(30) Priorität: 29.08.2018 IT 201800008220
(71) Anmelder: Laimer, Peter, 39011 Lana (BZ) (IT)
(72) Erfinder: Laimer, Peter, 39011 Lana (BZ) (IT)
(74) Vertreter: Ausserer, Anton

(57) **Zusammenfassung**

Die Erfindung betrifft ein Herstellungsverfahren eines präformierten Apfel- oder Birnenjungbaumes (1).

Das Verfahren umfasst erfindungsgemäß die folgenden Schritte:
a1) Pflanzen eines präformierten Apfel- oder Birnenjungbaumes (1),
oder
a2) Aufpfropfen/Aufpelzen eines präformierten Apfel- oder Birnenjungbaumes (1) auf einer Unterlage,
b) mechanische und/oder Hormonbehandlung des Leitastes (2), sodass nur auf einem Teil des Umfanges Äste (3) in einer ersten Wuchsperiode wachsen, wobei dieser Teil des Umfanges des Leitastes (2) einen Winkel alfa (α) von weniger als 100° umschließt,
c1) Auspflanzung des präformierten Apfel- oder Birnenbaumes wobei der Leitast (2) des Apfel- oder Birnenbaumes, (1) und der Untergrund einen Winkel a von 20° bis 80° umschließt,
oder
c2) Aufpelzen/Aufpfropfen des präformierten Apfel- oder Birnenbaumes (1) auf einer Unterlage, wobei Unterlage mit Leitast (2) des Apfel- oder die Birnenbaumes und der Untergrund einen Winkel a von 20° bis 80°,
d) Binden des Leitastes an einen ersten Draht (101), wobei der erste Teil des Leitastes (2) und der zweite Teil des Leitastes (2) einen Winkel b von mehr als 100° und weniger als 170° einschließen.

## Beschreibung

Die vorliegende Erfindung betrifft ein Verfahren zur Herstellung eines präformierten Jungbaums für Äpfel und Birnen und ein Verfahren zur Aufzucht einer Plantage / Obstgarten mit solchen Jungbäumen für Äpfel und Birnen.

Obstbäume sind für Plantagen schon seit langem bekannt. In der letzten Zeit stellt sich das Problem der mechanischen und /oder der autonomen Ernte des Obstes durch Maschinen. Um die autonome Ernte durch Maschinen zu erleichtern wird eine Anpassung der Pflanzen verlangt.

Unter präformierten Jungbaum für Äpfel und Birnen versteht man in der vorliegenden Beschreibung einen Obstbaum der durch Einfluss von Menschen gebildet d.h. formiert wird. Die Einflüsse können mechanische oder chemische Behandlungen sein.

Aus der WO 2008047297 ist ein Verfahren zur Herstellung von Obstbaumpflanzen mit zwei Leitästen bekannt, wobei das Verfahren die folgenden Schritte umfasst: Verpflanzung eines Wurzelstocks in den Boden zur Kultivierung und Kultivierung desselben;
auf den Wurzelstock wird Vermehrungsmaterial der Sorte gepfropft, zur Erzeugung von Trieben.

Auswahl und / oder Änderung der Position der Triebe mit dem Ziel, zwei einander gegenüberliegende Triebe auf jeder der Pflanzen zu erhalten, und
Kultivierung der Pflanzen bis zum Stadium, wo sie für die Obstproduktion ausgepflanzt werden können.

Das hier beschriebene Verfahren begrenzt den Endbenutzer in der Auswahl der Distanz der einzelnen Pflanzen und außerdem ist die Größe der Pflanzen zu groß, das begünstigt die Abdrift der chemischen Mittel, welche in der Landwirtschaft eingesetzt werden. Weiter führt diese Baumform zur größeren Breite einer Reihe der Obstbäume, das wiederum zum Wachstum eines größeren Anteils von Früchten zweiter Qualität führt: da sie nicht immer richtig der Sonne ausgesetzt sind, weil sie von Ästen oder Blättern bedeckt werden.

Aus der EP 2 944 186 ist ein Herstellungsverfahren von Obstbäumen mit einem Stamm bekannt, der etwa 70 cm oberhalb des Bodens um 90 Grad gebogen wird und dann im Wesentlichen horizontal verläuft, insbesondere für Äpfel und Birnen, ohne die Anwendung des Verfahrens auf die Verpflanzung von Obstbäumen auszuschließen. Dieses Herstellungsverfahren umfasst folgende Arbeitsphasen:
Aufpfropfen von Vermehrungsmaterial der zu vermehrenden Sorte auf eine Unterlage und pflanzen in einer Baumschule am Beginn der ersten Wuchsperiode, biegen um ca. 90° des Triebes welcher sich leicht oberhalb der Veredlungsstelle in der selben Wuchsperiode gebildet hat und anbinden an einem horizontal gespannten Draht am Ende der ersten Wachstumsperiode oder im Frühjahr der darauffolgenden Periode,
Auswahl mit Schnitt der kräftigen Triebe im Bereich des Buges, im Bereich des Endes des horizontalen Leitastes und in den Zwischenbereichen, um vier vertikale, unter sich ca. gleich beabstandete, Triebe , ca. zur Hälfte der zweiten Wachstumsperiode, zu erhalten,
Binden der einzelnen Fruchttriebe an einen horizontal gespannten Draht mit Beseitigung eventueller, im Bereich des Buges und längs des horizontalen Leitastes, in der Zwischenzeit gewachsener Triebe bis zum Ende der zweiten Wachtumsperiode,
Auspflanzen in die Anlage am Anfang der nächsten Wachstumsperiode, wobei der horizontale Leitast an einem ersten unteren gespannten Draht und die einzelnen vertikalen Triebe an einem zweiten oberen horizontalen Draht festgebunden werden.

Die Pflanze, welche durch dieses Verfahren hergestellt wird, weist einige wesentliche Probleme auf. Der Winkel von ungefähr 90° erlaubt es nicht, dass die Flüssigkeiten von der Wurzel in die anderen Teile der Pflanze regulär fließen. Das bedeutet, der Winkel von etwa 90 Grad führt zu einer starken, so genannten Scheitelpunktförderung im Bereich des Bugs, wodurch die Triebe in der Nähe des Bugs mit der Zeit immer stärker wachsen und die weiter entfernten Triebe verkümmern, und unbrauchbar werden. Außerdem liegt mit dem Winkel von ca. 90° die Distanz zwischen dem Boden und dem ersten metallischen Draht fest. Auf diese Art und Weise müssen die Maschinen, welche in der Obstplantage verwendet werden, an die Pflanze angepasst werden und nicht umgekehrt.

In der Veröffentlichung der Feno das Guyotsystem (https://www.fruitplant.it/de/anbausystem/) wird ein System beschrieben mit einem Baum mit einer waagrecht gebundenen Fruchtachse, ähnlich der Spaliererziehung im Weinbau. Der Baum hat bei der Lieferung bereits eine gewisse Neigung der Fruchtrute vorgegeben und kann einfach in der entgegengesetzten Richtung zur ursprünglichen Neigung in die Waagrechte gebunden werden. Des Weiteren ist die Fruchtachse nur einseitig mit Seitentrieben versehen, welche in Richtung zum Ende der Achse, länger werden um der natürlichen Neigung der Pflanzen nämlich der Triebförderung am Anfang der gebogenen Achse entgegenzuwirken.

Auch in diesem System wird ein Bug von im Wesentlichen 90° beschrieben. Dies verhindert wie in der vorgenannten Veröffentlichung, dass die Flüssigkeiten gut und frei im Baum fließen. Die auftretende Scheitelpunktförderung begrenzt auch in diesem Fall die Lebensdauer der Plantagen, wobei die anfangs etwas längeren Äste am Ende des Leitastes dies nur etwas hinauszögern, aber nicht verhindern können. Außerdem sind in diesem Fall auch die Äste im Wesentlichen in einer Linie angeordnet. Dies kann dazu führen, dass nicht alle Äste mit genügend Nährstoffen versorgt werden, da sie durch dieselben Kanäle versorgt werden müssen. Der Produktionsprozess in der Baumschule dauert hier mindestens zwei Jahre und schließt eine Verwendung des Pflanzmaterials bereits unmittelbar nach dem Produktionsbeginn für jegliche andere Zwecke aus, was in der heutigen schnelllebigen Zeit nicht annehmbare Bedingungen sind.

Aus der CN 108 064 574 ist ein Verfahren zur Aufzucht von Kirschbäumen beschrieben. Nach dem zweiten Jahr der Aufzucht werden die Kirschbäume in die Plantage verpflanzt. Der erste und der zweite und usw. Baum werden miteinander verbunden indem sie gegenseitig aufgepfropft werden. Damit bilden mehrere Bäume zusammen eine stabile verwachsene Struktur. So soll verhindert werden, dass der einzelne Baum sehr stark wächst, was bei Kirschenbäumen eine Vielzahl von Nachteilen aufweist. Durch das Zusammenwachsen der Bäume soll die ApikalDominanz der einzelnen Pflanze ausgeschaltet werden, was zu einer Vielzahl von gleich stark wachsenden Trieben führen soll. Das dort beschriebene Verfahren ist sehr zeitaufwändig und muss zur Gänze in der Plantage/Obstgarten durchgeführt werden.-Dadurch dauert die Erstellung einer Neuanlage mehrere Jahre, die Folge sind Ernteausfälle in den Jahren der Aufzucht. Der spitze Winkel zum Boden(30°-45°) auf den die Stämme der Obstbäume bei diesem Verfahren nieder gebunden werden, erschwert in besonderem Maße die mechanische Unkrautbekämpfung. Die heute üblichen Geräte die mit Hilfe von vertikal rotierenden Plastikfäden das Gras einkürzen, führen bei stark geneigten Stämmen zu starken Verletzungen der Rinde, wodurch Krankheitserreger in die Pflanze eindringen können.

Sollte es bei diesem Verfahren zur Erkrankung einer einzelnen Pflanze mit einem Virus oder Phytoplasma oder einem anderen durch den Saftstrom wandernden Krankheitserreger kommen, ist unweigerlich mit dem Ausfall einer ganzen Reihe zu rechnen, da jede Pflanze einer Reihe mit der nächsten verbunden ist.

Die Aufgabe der vorliegenden Erfindung ist ein Verfahren zur Aufzucht eines präformierten Apfel- oder Birnenbaumes und ein Verfahren zur Aufzucht einer Plantage/Obstgarten umfassend einen präformierten Jungbaums gezüchtet nach dem kennzeichnenden Teil des Anspruches 1 und/oder ein Apfel- oder Birnenbaumes gezüchtet nach den Merkmalen des Verfahrens eines präformierten Jungbaum nach dem kennzeichnen Teil des Anspruches 1, welche die oben genannten Probleme löst.

Ein Herstellungsverfahren eines Apfel- oder Birnenbaumes umfasst die folgenden Schritte:
a1)pflanzen eines präformierten Apfel- oder Birnenbaumes in bevorzugter Weise in einer Baumschule
   oder
a2)aufpfropfen/aufpelzen eines präformierten Apfel- oder Birnenbaumes auf einer Unterlage in bevorzugter Weise in einer Baumschule,
b)mechanische Stimulierung und/oder Hormonbehandlung des vertikal nach oben wachsenden Triebes zur Förderung von Seitentrieben bei gleichzeitiger Eliminierung der unerwünschten Seitentriebe, sodass nur auf einem Teil des Umfanges des Leitastes Seitentriebe wachsen. Dies geschieht in regelmäßigen Abständen mindestens bevorzugter Weise während einer gesamten Wachstumsperiode bis der entstandene Leitast eine Höhe von 1,5 - 1,7 m vom Boden erreicht wobei die Seitentriebe in einem Winkel alfa α von weniger als 180° angeordnet sind, in einer bevorzugten Ausführungsform weniger als 100°.

Durch die kontinuierliche Stimulation des Wachstums und das dadurch fortwährende Wachstum des Jungbaums entstehen im unteren Baumbereich längere Seitentriebe als im oberen Baumbereich, was so erwünscht ist, außerdem beginnt die Stimulation der Verzweigung des Leitastes bereits bei Vegetationsbeginn, sobald dieser eine Höhe von etwa 10cm erreicht. Bei einer üblichen Veredlungshöhe von 10- 30 cm führt das zu den ersten Seitentrieben, die ab einer Höhe von etwa 30 cm über dem Boden bevorzugt ab 35 cm formiert werden. Der so präformierte Jungbaum kann durch Rodung aus der Baumschule entnommen werden.

In einem Schritt b1 wird der präformierten Apfel- oder Birnenbaums aus der Baumschule entnommen.

Unter Rodung wird z.B. auch die Entnahme mittels eines Rodungspfluges verstanden oder in einer anderen Form z.B. durch schneiden.
c1)Auspflanzung des präformierten Apfel- oder Birnenbaumes, in der Obstplantage, wobei der Leitast des Apfel- oder Birnenbaumes und der Untergrund einen Winkel a von 20° bis 80° insbesondere 50° bis 60 °einschließen,
   oder
c2)aufpelzen/aufpfropfen des Apfel- oder Birnenbaumes auf eine Unterlage, wobei die Unterlage mit Leitast des Apfel- oder Birnenbaumes und der Untergrund einen Winkel a von 20° bis 80°, insbesondere 50° bis 60° einschließen,
d)binden des Leitastes im Laufe des Pflanzjahres durch Binden an einem ersten Draht,
e)biegen des Leitastes im Laufe des Pflanzjahres durch Binden an einem zweiten Draht,
wobei der erste Teil des Leitastes unter dem ersten Draht und der zweite Teil des Leitastes oberhalb der Bindestelle an den ersten Draht einen Winkel b von mehr als 100° und weniger als 170° einschließen, bevorzugt über 140° und vorteilhafter Weise zwischen 145 und 150°. Dieser erste Draht wird bevorzugt auf einer Höhe von 50 cm über dem Boden gespannt.

In bevorzugter Weise wird in einem weiteren Verfahrensschritt, später aber immer noch innerhalb des Pflanzjahres der Leitast ein zweites mal gebogen, durch Binden an einem dritten Draht, wobei der zweite Teil des Leitastes und der Teil des Leitastes oberhalb des zweiten Drahtes einen Winkel c von mehr als 100° und weniger als 170° einschließen, bevorzugt über 140° und vorteilhafter Weise zwischen 145° und 150°. Dieser zweite Draht wird bevorzugt etwa 65 cm über dem Boden gespannt.

In einer erfindungsgemäßen Ausführungsform in einem weiteren Verfahrensschritt, wird immer noch innerhalb des Pflanzjahres der Leitast durch Binden an einen vierten Draht derart nach oben gebogen, dass die Spitzes des Baumes im Wesentlichen vertikal zum Boden/Untergrund steht. Dieser dritte Draht wird bevorzugt 1,2m über dem Boden gespannt.

Die Anzahl der Biegungen und Anbindungen an Drähte kann in Funktion von externen Einflüssen und Bodenverhältnissen und Art des präformierten Jungbaumes variieren.

In bevorzugter Weise werden die Seitentriebe im Verfahrensschritt b) geradlinig gezogen d.h., dass sie über den gesamten Teil des Umfanges des Leitastes angeordnet sind. Der Austritt der Seitentriebe aus dem Leitast erfolgt im Wesentlichen im Winkel von 90° zur Tangente des Umfanges des Leitastes und sollte auf dem gesamten Teil des Umfanges erfolgen, damit die Seitentriebe aus unterschiedlichen Adern, Kanälen des Baumes Flüssigkeiten und Nährstoffe beziehen. Durch Hochbinden sind die Äste im Wesentlichen vertikal, die ursprünglichen Austrittspunkte werden dadurch nicht beeinflusst.

Nach dem Verpflanzen des Jungbaums in der Obstplantage und die Fixierung des Leitastes an den Drähten werden die Seitentriebe ebenfalls an den Drähten fixiert. Dies geschieht so, dass die Äste zwar vertikal nach oben gebunden werden, die unterschiedlichen Austrittspunkte aber nicht verbogen werden.

Die präformierten Bäume/Baumpflanzen werden dann gepflanzt oder die präformierten Jungbäume werden dann aufgezogen, nachdem sie aufgepelzt /gepfropft nach den Merkmalen der Erfindung in einer Plantage/Obstgarten in einer Reihe und mit einem Winkel geneigt gegenüber dem Untergrund oder gegenüber dem Baumstumpf auf dem sie aufgepelzt/gepfropft werden. Dieser Winkel ist ein Winkel der größer ist als 90° und weniger als 170 °gegenüber der vertikalen Achse in bevorzugter Weise über 140° und vorteilhafter Weise zwischen 145° und 150°.

Auf diese Weise, auch wenn die Seitentriebe nur auf einem Teil des Umfangs des Leitastes angeordnet sind, füllt die Fläche, die durch die hochgebundenen Seitentriebe gebildet wird die gesamte Fläche, die durch die Reihe der Bäume gebildet wird. Außerdem wird durch die längeren Seitentriebe im unteren Baumbereich eine mehr oder weniger gleich hohe Wand gebildet, eine ganze Fläche abgedeckt von Seitentrieben, auf welchen das Obst wächst. Normalerweise kann man so bereits im Jahr nach dem Pflanzjahr eine nicht unerhebliche Ernte einbringen. Die verschiedenen Biegewinkel führen den Baum sanft an die Erziehungsform und es kommt zu keiner Scheitelpunktförderung, durch die Beibehaltung der ApikalDominanz, was durch das Hochbinden der Spitze des Leitastes, und das ständige Ansteigen desselben bewirkt wird und es können langlebige Obstplantagen mit ausgewogenem Wachstum erzeugt werden, wie es bei anderen Formen nicht möglich ist. Es ist nicht nötig eine erste Fläche ohne Obst im unteren Bereich zu haben. Auf diese Art und Weise stellt man eine Fläche her, auf der dann die Früchte angeordnet sind mit einer sehr geringen Breite der Pflanzenreihe. Das Obst kann auf diese Art und Weise sehr gut wachsen, da es der Sonne ausgesetzt ist. Außerdem ist das Obst leichter durch eine Maschine zu ernten, da es nicht durch Äste und/oder wenigen Äste bedeckt ist. Auch wenn die Gesamtmenge des Obstes auf diese Art und Weise reduziert ist, nimmt die Anzahl der Früchte erster Qualität nicht ab. Daher werden die Kosten der Ernte gesenkt, obwohl man die Anzahl der Früchte erster Qualität beibehält und/oder diese erhöht in Bezug auf die Fläche der Plantage/Obstgarten .

Die Nutzung einer größeren Oberfläche, durch Ausnutzung auch der tiefer ansetzenden Seitentriebe und so auch der Fläche in der Nähe des Bodens ist insbesondere nützlich wenn zum Ernten Maschinen eingesetzt werden. Für diese Maschinen ist es unwichtig, ob die Furcht sich in der Nähe des Boden befindet oder in der Höhe. Hingegen für eine Person, welche Obst erntet, ist es, sehr wohl ein Unterschied, da sich die Person beugen muss und dies bringt Rückenprobleme mit sich. Daher wurden bisher die Pflanzen so aufgezogen, dass sie in Bodennähe keine Seitentriebe mit Obst hatten.

Außerdem erlaubt ein Winkel von über 90°und weniger als 170° einen besseren Fluss der Flüssigkeiten innerhalb eines präformierten Jungbaumes.

In einer bevorzugten Ausführungsform weist der präformierte Jungbaum mehrere Biegungen auf mit unterschiedlichen Neigungswinkeln. Diese Winkel werden durch das Anbinden des präformierten Jungbaumes an Draht insbesondere Metalldraht erreicht.

Durch ein erfindungsgemäßes Verfahren kann eine Plantage hergestellt werden, welche erfindungsgemäße präformierten Jungbäume umfasst, wobei das Verfahren die folgenden Schritte umfasst:
- Pflanzen einer erfindungsgemäßen präformierten Jungbaumes im Boden, wobei der Winkel zwischen der vertikalen Achse und der Seite des Leitastes welche keine Seitentrieb trägt >100°, bevorzugt zwischen 140° und 150° und weniger als 170° ist,
oder aufziehen eines präformierten Jungbaumes, welcher auf einem vorhandenen Baumstumpf/Unterlage aufgepelzt/aufgepfropft wird, wobei aus dem präformierten Jungbaum seitlich erste Seitentriebe, welche direkt aus dem Leitast austreten, bereits vorhanden sind, und wobei Seitentriebe des Leitastes nur innerhalb eines Teiles des Umfangs des Leitastes gezogen werden und in einer bevorzugten Ausführungsform oberhalb einer Höhe von zumindest 1,50 m bevorzugt 1,70 m können die Äste natürlich ohne menschliches Eingreifen wachsen, da dieses Wachstum erst nach dem Verfahrensschritt b erfolgt. In diesem Bereich wird keine Präformation durchgeführt.

Der Teil des Umfangs umfasst vorteilhafterweise ein Winkel von 180° in bevorzugter Weise 100°. Der Winkel der zwischen der gegenüberliegenden Seite des Leitastes, an der die Seitetriebe wachsen, liegt, beträgt der Winkel zur vertikalen Achse mehr als (>)100° und weniger als 170° in bevorzugter Weise zwischen 140-150°.

In einem bevorzugten Verfahren werden die an einander gereihten Pflanzen an Drähte gebunden zum Beispiel Metalldrähte, um die Neigung des Leitastes zu variieren. Vorteilhafterweise ist der Winkel zwischen der gegenüberliegenden Seite des Leitastes an der die Triebe wachsen zur vertikalen Achse mehr als (>)125° und weniger als 170°bevorzugter Weise zwischen 130°-140°. In bevorzugter Weise werden die aneinandergereihten präformierten Jungbäume an einen Draht gebunden zum Beispiel einen Metalldraht, wobei deren Neigung variiert wird, vorteilhafter Weise bei der zweiten Anbindung ist der Winkel, zwischen der gegenüberliegenden Seite des Leitastes an der die Triebe wachsen zur vertikalen Achse mehr als (>)150° und weniger als 170°und bevorzugter Weise zwischen 155 und 165°. Weitere Merkmale und Details der erfindungsgemäßen präformierten Jungpflanze und ein Verfahren zur Aufzucht einer Plantage mit denselben präformierten Jungbäumen für Äpfel und Birnen in einer nicht begrenzenden Ausführungsform der Erfindung werden folgend im Detail in der folgenden Beschreibung mit Bezugnahme auf die beiliegenden Figuren beschrieben:
die Figur 1 stellt eine erfindungsgemäße Baumpflanze in einer seitlichen Ansicht dar,
die Figur 2 zeigt einen erfindungsgemäße präformierten Jungbaum in einer Ansicht von oben,
die Figur 3 zeigt eine Plantage mit einer Serie von erfindungsgemäßen Baumpflanzen, die Figur 4a,zeigt den präformierte Jungbaum

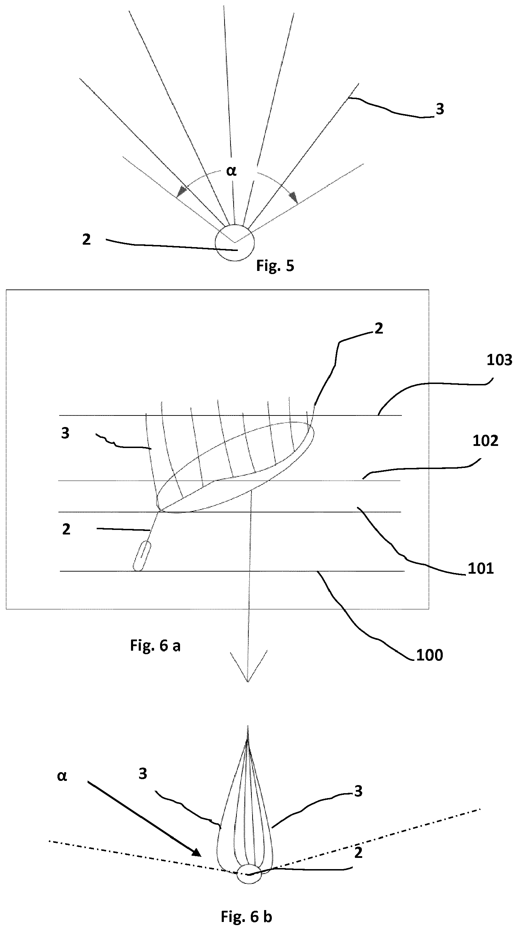

die Figuren 4b,4c,4d und 4e zeigen die einzelnen Schritte eines erfindungsgemäßen Herstellungsverfahrens der Obstplantage , die Figur 5 zeigte eine Ansicht von oben einen präformierte Jungbaum,
die Figur 6a zeigt in einer Seitenansicht einen präformierte Jungbaum in der Obstplantage und die Figur 6b zeigt eine Ansicht von oben der Figur 6a mit Anordnung der Äste.

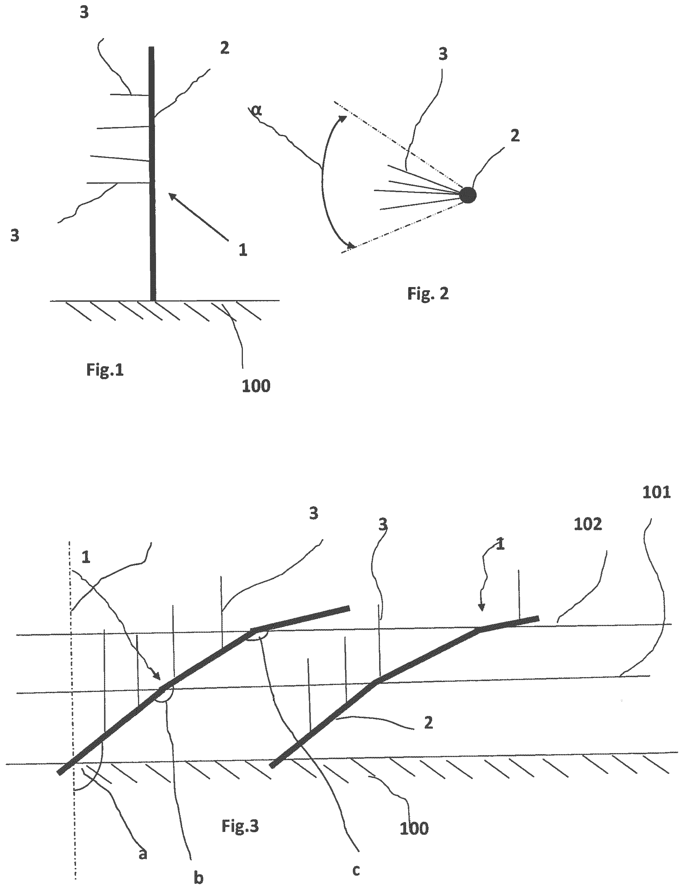

In der Figur 1 ist mit der Bezugsziffer 1 eine präformierten Jungpflanze, für Apfel und Birnen bezeichnet, welche durch einen Leitast 2 gebildet wird und welche in den Boden 100 verpflanzt ist und dieser präformierte Jungbaum wird zum Beispiel in einer Baumschule aufgezogen. Während der Aufzucht werden die Ansätze der Äste/Seitentriebe 3, die vom Leitast 2 auf die Art und Weise wachsen, derart geschnitten ,stimuliert und gezogen, dass sie nur in einem Teil des Umfanges um den Leitast angeordnet sind, wobei der Teil einen Winkel alfa α aufweist und wobei alfa α kleiner als 180° ist, vorzugsweise kleiner als 100° ist, wie in Figur 2 dargestellt. Diese Aufzucht erfolgt in einer ersten Wachstumsperiode, die in bevorzugter Weise ein Jahr dauert, wenn das Zuchtmaterial durch Aufpfropfen/Aufpelzen des Apfel- oder Birnenbaumes 1 auf einer Unterlage erfolgt. Durch mechanische und/oder Hormonbehandlung des Leitastes 2, sodass nur auf einem Teil des Umfanges Seitentriebe 3 in einer ersten Wuchsperiode wachsen, beträgt dieser Teil einen Winkel alfa α von weniger als 180°, in einer bevorzugten Ausführungsform weniger als 100°.

Diese Zucht/Aufzucht (Präformation) wird in bevorzugter Weise bis zu einer Höhe von 1,50 m vom Boden durchgeführt in bevorzugter Weise bis zu 1,70 m vom Boden, da der Obstbaum diese Höhe normalerweise in der ersten Wachstumsperiode erreicht. Diese erfolgt in bevorzugter Weise in einer Baumschule. Da im erfindungsgemäßen Verfahren für die Herstellung eines präformierten Jungbaums nur ein Jahr benötigt wird, ist die Baumschule flexibler und kann sich schneller an neue Sorten anpassen und muss auch nicht so lange warten, um ein Einkommen zu erzielen.

Der präformierte Jungbaum 1 wird nach Beendigung der Aufzucht aus der Baumschule genommen, also gerodet und in die Plantage, Obstgarten gebracht, wo er dann gepflanzt wird.

Die Aufzucht des präformierten Jungbaums kann auch in Plantagen, Obstgarten durchgeführt werden, die bereits vorhanden sind, auch bei vorhandenen Baumstämmen/Baumstümpfen. Zum Beispiel werden die präformierte Jungbäume 1 auf Baumstümpfe/Unterlagen der Art M9 aufgepelzt/ veredelt. Auch wenn die Baumstämme der Art M9 besondere Aufmerksamkeit verlangen und sensibel gegen schlechte Witterungsbedingungen sind, verlangen sie außerdem große Mengen an Wasser, da sie sehr produktiv sind. Für die erfindungsgemäßen Pflanzen könnten auch Baumstümpfe, verwendet werden, die bereits existieren, da dies weniger Ressourcen verbrauchen.

In vorteilhafter Weise werden die präformierten Jungbäume 1 nach einem Verfahren verpflanzt, indem der Leitast 2 in den Boden gesetzt wird oder die Pflanze wird auf einer bestehenden Pflanze aufgezogen, zum Beispiel auf einem Baumstumpf, welcher die Merkmale einer erfindungsgemäßen Pflanze aufweist, wobei er einen Winkel a zwischen der gegenüberliegenden Seite der Äste des Baumstumpf 2 einschließt, welcher zwischen 20° und 80° ist, vorteilhafter Weise zwischen 50° und 60°.

Es sollte zumindest ein zweiter erfindungsgemäßer präformierter Jungbaum in den Boden gepflanzt werden, oder aufgepelzt oder aufgepfropft auf einer bereits bestehenden Pflanze werden, zum Beispiel einem Leitast mit denselben Merkmalen eines erfindungsgemäßen präformierten Jungbaums zum Beispiel mit einem Baumstumpf, welche einen Winkel zwischen der vertikalen Achse und der gegenüberliegenden Seite der Seitentriebe 3 einschließen welcher >100° vorteilhafter Weise zwischen 140° und 150° ist und wobei dieser präformierte Jungbaum im wesentlichen mit dem ersten präformierte Jungbaum aufgereiht ist.

Durch Hochbinden sind die Äste im Wesentlichen vertikal, die ursprünglichen Austrittspunkte werden dadurch nicht beeinflusst und beibehalten. Der präformierte Jungbaum weist eine minimale seitliche Ausdehnung in Bezug auf die Reihe auf, die durch die Pflanzen gebildet wird.

In einem bevorzugten Verfahren werden die Pflanzen aufgereiht und an einem Draht gebunden, zum Beispiel einem Metalldraht 101,102,103, wobei die Neigung des Leitastes geändert wird, in bevorzugter Weise bei der ersten Anbindung ist der Winkel, welchen der Baum auf der gegenüberliegenden Seite Äste 3 zwischen den beiden Teilen des Leitastes einschließt, über 145° in bevorzugter Art und Weise zwischen 150° und 160°.

Der Teil über 1,5 m bevorzugt über 1,7 m kann eine Baumkrone bilden und diese kann in die Höhe vertikal zum Boden gebogen werden.

In einer bevorzugten Variante des Herstellungsverfahren eines Apfel- oder Birnenbaumes 1 umfasst dieser die folgenden Schritte:
a1)pflanzen eines präformierten Jungbaums für Äpfel- oder Birnen 1, in bevorzugter Weise in einer Baumschule
   oder
a2)aufpfropfen/aufpelzen eines präformierten Jungbaums für Äpfel oder Birnen 1 auf einer Unterlage zum Beispiel in bevorzugter weise in einer Baumschule.

Wenn der Apfel- oder Birnenbaum 1 aufgepfropft wird, kann man den Umstand ausnutzen, dass die Unterlage bestimmte Wachstums- und Ertragseigenschaften an die aufgepfropfte Sorte weitergibt, die sonst nicht gegeben wären.

Im zweiten Schritt b) werden mechanische und/oder Hormonbehandlungen am Leitast 2 durchgeführt. Auf diese Art und Weise wachsen in einer ersten Wuchsperiode nur auf einem Teil des Umfanges Seitentriebe, wobei dieser Teil einen Winkel alfa α von weniger als 180° umfasst, in einer bevorzugten Ausführungsform weniger als 100°.

Die Hormonbehandlung kann mittels Hormone zum Beispiel Gibberelline aus der Gruppe der Phytohormone, Cytokinine und Auxinhemmererfolgen.

Diese Hormonbehandlung und/oder die mechanische Behandlung erfolgt bevorzugt in Kombination mit Düngemittel um das Wachstum zu stimulieren.

Bei der mechanischen Bearbeitung werden die Triebe insbesondere die Triebspitze / die Spitze des Leitastes bearbeitet. Das heißt der Trieb des Leitastes wird leicht beschädigt und dadurch wird verhindert oder vermindert die Ausschüttung von Hormonen, die eine Bildung von Seitentriebe hemmen. Außerdem werden die Seitentriebe nur im erwünschten Teilbereich des Umfanges des Leitastes d.h. innerhalb einem Teil des Umfanges Seitentrieb 3 präformiert dieser Teil umfasst einen Winkel alfa α von weniger als 180°, in einer bevorzugten Ausführungsform weniger als 100°.

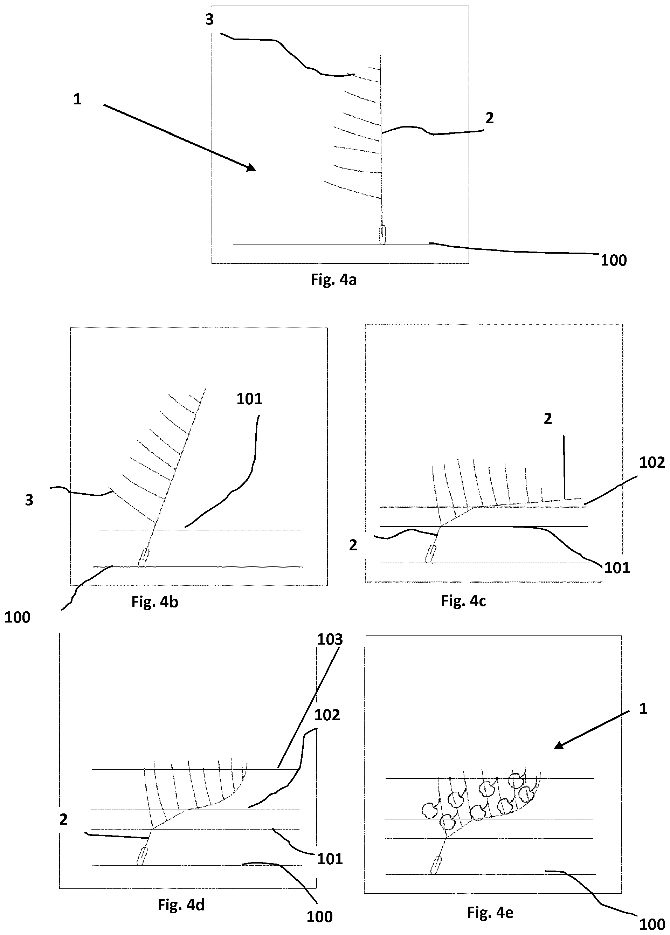

In der Figura 4a ist ein Baum 1 dargestellt der eine solche Behandlung erfahren hat. Durch die Behandlung sind die Seitentriebe 3 aber nicht alle gereiht, sondern über den gesamten Teilumfang des Leitastes 2 innerhalb des Winkels alfa α verteilt.

In einem weiterem Schritt c1 erfolgt die Auspflanzung des Apfel- oder Birnenbaumes 1, wobei der Leitast 2 des Apfel- oder Birnenbaumes 1 und der Untergrund/Boden 100 einen Winkel a von 20° bis 80° insbesondere 50° bis 60°einschließen,
oder
In Alternative zu c1)kann der Schritt wie folgt erfolgen: c2)aufpelzen/aufpfropfen des Apfel- oder Birnenbaumes auf einer Unterlage wobei Unterlage mit Leitast des Apfel- oder Birnenbaumes 1 und der Untergrund 100 einen Winkel a von 20° bis 80°, insbesondere 50° bis 60° einschließen.

In einem weiterem Schritt d) erfolgt das binden des Leitastes 2 durch Binden an einen ersten Draht 101, durch dieses Binden wird der Winkel a mit dem Untergrund gewährleistet.

Dies erfolgt vorteilhafter Weise in einer Höhe von 30, 40, besser 50cm cm vom Boden/Untergrund 100. Ein Baum 1 nach den Schritt c1 oder c2 und d ist in Figura 4b dargestellt.

Durch Biegen und Binden des Leitastes 2 an einen zweiten Draht 102 wobei der erste Teil des Leitastes 2 und der zweite Teil des Leitastes einen Winkel b von mehr als 100° und weniger als 170° einschließen bevorzugt über 140° und vorteilhafter Weise, zwischen 145° und 150°. Ein Baum 1 nach den Schritt c1 oder c2 und d ist in Figur 4c dargestellt.

In bevorzugter Weise wird in einem weiteren Verfahrensschritt, in dem der Leitast 2 ein zweites Mal gebogen wird, durch binden an einen dritten Draht 103, wobei der zweiten Teil des Leitastes und der dritte Teil des Leitastes einen Winkel c von mehr als 100° und weniger als 170° einschließen bevorzugt über 140° und vorteilhafter Weise zwischen 145 und 150°. Dies ist in Figur 4d dargestellt. Durch das Binden im diesem Verfahrensschritt kann gleichzeitig die Spitze des Leitastes/ die Triebspitze nach oben gebogen werden. Durch das Biegen und Binden wird dieSpitzes des Baumes im Wesentlichen vertikal zum Boden/Untergrund 100 stehen. In Figur 4e wird ein erfindungsgemäßer Baum 1 dargestellt der Obst trägt. Das nach oben Biegen der Spitze des Leitastes/ der Triebspitze kann aber auch in einem anderen Schritt auch unabhängig davon durchgeführt werden.

Es können mehrere Schritte zum Biegen oder Binden an Drähte dem Erfindungsgemäßen Verfahren hinzugefügt werden.

Die vorangehend beschriebenen Varianten dienen, lediglich dem besseren Verständnis der Struktur, der Funktionsweise und der Eigenschaften der vorgestellten Lösung; sie schränken die Offenbarung nicht etwa auf die Ausführungsbeispiele ein. Die Fig. sind schematisch, wobei wesentliche Eigenschaften und Effekte zum Teil deutlich vergrößert dargestellt sind, um die Funktionen, Wirkprinzipien, technischen Ausgestaltungen und Merkmale zu verdeutlichen. Dabei kann jede Funktionsweise, jedes Prinzip, jede technische Ausgestaltung und jedes Merkmal, welches / welche in den Fig. oder im Text offenbart ist/sind, mit allen Ansprüchen, jedem Merkmal im Text und in den anderen Fig., anderen Funktionsweisen, Prinzipien, technischen Ausgestaltungen und Merkmalen, die in dieser Offenbarung enthalten sind oder sich daraus ergeben, frei und beliebig kombiniert werden, so dass alle denkbaren Kombinationen der beschriebenen Lösung zuzuschreiben sind. Dabei sind auch Kombinationen zwischen allen einzelnen Ausführungen im Text, das heißt in jedem Abschnitt der Beschreibung, in den Ansprüchen und auch Kombinationen zwischen verschiedenen Varianten im Text, in den Ansprüchen und in den Fig. umfasst. Die vorstehend erläuterten Vorrichtungs- und Verfahrensdetails sind zwar im Zusammenhang dargestellt; es sei jedoch darauf hingewiesen, dass sie auch unabhängig voneinander sind und auch frei miteinander kombinierbar sind. Die in den Fig. gezeigten Verhältnisse der einzelnen Teile und Abschnitte hiervon zueinander und deren Abmessungen und Proportionen sind nicht einschränkend zu verstehen. Vielmehr können einzelne Abmessungen und Proportionen auch von den gezeigten abweichen. Auch die Ansprüche limitieren nicht die Offenbarung und damit die Kombinationsmöglichkeiten aller aufgezeigten Merkmale untereinander. Alle aufgezeigten Merkmale sind explizit auch einzeln und in Kombination mit allen anderen Merkmalen hier offenbart.

## Patentansprüche

1. Herstellungsverfahren eines präformierten Apfel- oder Birnenjungbaumes (1), umfassend die folgenden Schritte:
a1)Pflanzen eines präformierten Apfel- oder Birnenjungbaumes (1),
oder
a2)Aufpfropfen/Aufpelzen eines präformierten Apfel- oder Birnenjungbaumes (1) auf einer Unterlage,
b)mechanische und/oder Hormonbehandlung des Leitastes (2), sodass nur auf einem Teil des Umfanges Äste (3) in einer ersten Wuchsperiode wachsen, wobei dieser Teil des Umfanges des Leitastes (2) einen Winkel alfa (α) von weniger als 100° umschließt,
c1)Auspflanzung des präformierten Apfel- oder Birnenbaumes wobei der Leitast (2) des Apfel- oder Birnenbaumes (1) und der Untergrund einen Winkel a von 20° bis 80° umschließt,
oder
c2)Aufpelzen/Aufpfropfen des präformierten Apfel- oder Birnenbaumes (1) auf einer Unterlage, wobei Unterlage mit Leitast (2) des Apfel- oder die Birnenbaumes und der Untergrund einen Winkel a von 20° bis 80°,
d)Binden des Leitastes an einen ersten Draht (101), wobei der erste Teil des Leitastes (2) und der zweite Teil des Leitastes (2) einen Winkel b von mehr als 100° und weniger als 170° einschließen.

2. Herstellungsverfahren eines Apfel- oder Birnenbaumes (1) nach Anspruch 1, **dadurch gekennzeichnet, dass** nach Schritt d) der Schritt e) erfolgt: Biegen und Binden des Leitastes (2) an einen zweiten Draht wobei der erste Teil des Leitastes (2) unterhalb des ersten Drahtes (101) und der zweite Teil des Leitastes (2) oberhalb des ersten Drahtes einen Winkel b von mehr als 100° und weniger als 170° einschließen.

3. Herstellungsverfahren eines Apfel- oder Birnenbaumes (1) nach Anspruch 1, **dadurch gekennzeichnet, dass** die Schritte a und b in einer Baumschule erfolgen und nach b der Schritt b1 erfolgt:
b1) Entnahme des präformierten Apfel- oder Birnenbaumes aus der Baumschule.

4. Herstellungsverfahren eines Apfel- oder Birnenbaumes (1) nach Anspruch 1,2 oder 3, **dadurch gekennzeichnet, dass** der Winkel alfa α weniger als 45° beträgt,
dass der Winkel a zwischen 50° und 60° liegt und der Winkel b zwischen 145° und 150° ist.

5. Herstellungsverfahren eines präformierten Apfel- oder Birnenbaumes (1) nach einem der vorhergehenden Ansprüche, **dadurch gekennzeichnet**, folgende Schritte zu umfassen:
in einem weiterem Schritt wird der Leitast (2) ein zweites Mal durch Binden an einen dritten Draht (103) gebogen, wobei der zweite Teil des Leitastes unterhalb des zweiten Drahtes (102) und der dritte Teil des Leitastes oberhalb des zweiten Drahtes (102) einen Winkel c von mehr als 100° und weniger als 170° einschließen, bevorzugt über 140° und vorteilhafter Weise zwischen 145° und 150°.

6. Herstellungsverfahren eines präformierten Apfel- oder Birnenbaumes (1) nach Anspruch 3, **dadurch gekennzeichnet dass** der Leitast (2) durch Binden an den dritten Draht (103) derart nach oben gebogen wird, dass die Spitzes des präformierten Apfel- oder Birnenbaumes (1) im Wesentlichen vertikal zum Boden/Untergrund (100) steht.

7. Herstellungsverfahren eines präformierten Apfel- oder Birnenbaumes (1) nach einem der vorhergehenden Ansprüche, **dadurch gekennzeichnet, dass** die mechanische Behandlung des Leitastes aus der teilweise Beschädigung des Triebes des Leitastes (2) besteht.

8. Herstellungsverfahren eines präformierten Apfel- oder Birnenbaumes (1) nach einem der vorhergehenden Ansprüche, **dadurch gekennzeichnet dass** die Hormonbehandlung durch Gibberelline aus der Gruppe der Phytohormone, Cytokinine und Auxinhemmer erfolgt.

9. Herstellungsverfahren eines präformierten Apfel- oder Birnenbaumes (1) nach einem der vorhergehenden Ansprüche, **dadurch gekennzeichnet dass** der Schritt b1) durch Rodung mittels eines Rodung-Pfluges erfolgt.

10. Herstellungsverfahren eines präformierten Apfel- oder Birnbaums (1) nach einem der vorhergehenden Ansprüche, **dadurch gekennzeichnet dass** die präformierten Apfel- oder Birnenbaumes (1) Baumstümpfe/Unterlagen der Art M9 aufgepelzt/ veredelt werden.

11. Herstellungsverfahren eines präformierten Apfel- oder Birnenbaumes (1) nach einem der vorhergehenden Ansprüche, **dadurch gekennzeichnet, dass** die ersten Seitentrieben (3) ab einer Höhe von etwa 30 cm über dem Boden bevorzugt ab 35 cm formiert werden.

12. Herstellungsverfahren eines präformierten Apfel- oder Birnenbaumes (1) nach einem der vorhergehenden Ansprüche, **dadurch gekennzeichnet dass** die ursprünglichen Austrittspunkte durch das Hochbinden von Seitentrieben (3) nicht beeinflusst werden.

13. Herstellungsverfahren eines präformierten Apfel- oder Birnenbaumes (1) nach einem der vorhergehenden Ansprüche, **dadurch gekennzeichnet dass** die mechanische und/oder Hormonbehandlung des Leitastes (2) gleichzeitig mit einer Düngung erfolgt.

14. Apfel- oder Birnbaum (1) hergestellt durch einem Verfahren gemäß einem der vorhergehenden Ansprüche.
